# EUROPÄISCHE PATENTSCHRIFT

(11) **EP 1 508 565 B1**
(45) Veröffentlichungstag und Bekanntmachung des Hinweises auf die Patenterteilung: **13.08.2008**
(21) Anmeldenummer: 04027540.6
(22) Anmeldetag: 24.11.1998
(51) Int. Cl.: C12P 41/00, C12P 13/00

(54) **Verfahren zur Herstellung von optisch aktiven Aminoalkoholen**
Process for the preparation of optically active aminoalcohols
Procédé de préparation d' aminoalcools optiquement actifs

(30) Priorität: 27.11.1997 CH 273997; 03.12.1997 CH 278197; 21.01.1998 CH 1331998; 27.03.1998 CH 7231998; 07.10.1998 EP 98118895
(43) Veröffentlichungstag der Anmeldung: 23.02.2005
(62) Teilanmeldung aus: 04002913.4
(73) Patentinhaber: Lonza AG, 3930 Visp (CH)
(72) Erfinder: Berchtold, Katja, 3937 Visp (CH); Roduit, Jean-Paul, 1950 Sion (CH); Bernegger-Egli, Christine, Dr., 3985 Münster (CH); Urban, Eva, Maria, 3912 Termen (CH); Petersen, Michael, Dr., 79540 Lörrach (DE)
(74) Vertreter: Winter, Brandl, Fürniss, Hübner Röss, Kaiser, Polte Partnerschaft Patent- und Rechtsanwaltskanzlei

(56) Entgegenhaltungen:
- EP-A- 0 434 450
- EP-A- 0 878 548
- WO-A-93/17020
- WO-A-95/21161
- WO-A-97/45529
- WO-A-99/19327
- EVANS C T ET AL: "POTENTIAL USE OF CARBOCYCLIC NUCLEOSIDES FOR THE TREATMENT OF AIDS CHEMO-ENZYMATIC SYNTHESES OF THE ENANTIOMERS OF CARBOVIR" JOURNAL OF THE CHEMICAL SOCIETY, PERKIN TRANSACTIONS 1, CHEMICAL SOCIETY. LETCHWORTH, GB, Nr. 5, 1992, Seiten 589-592, XP002089871 ISSN: 0300-922X
- CAMPBELL, JEFFREY A. ET AL: "Chirospecific Syntheses of Precursors of Cyclopentane and Cyclopentene Carbocyclic Nucleosides by [3 + 3]-Coupling and Transannular Alkylation" J. ORG. CHEM., Bd. 60, Nr. 14, 1995, Seiten 4602-4616, XP002041314
- N. KATAGIRI ET AL.: "Deamination of 9-(hydroxymethylated cycloalkyl)-9H-adenines (carbocyclic adenine nucleosides) by adenosine deaminase: Effect of the high-pressure upon deamination rate and enantioselectivity" NUCLEOSIDES & NUCLEOTIDES., Bd. 15, Nr. 1-3, 1996, Seiten 631-647, XP002124310 MARCEL DEKKER, INC., US ISSN: 0732-8311
- J.R. MALPASS ET AL.: "Reaction of Chlorosuphonyl Isocyanate with 1,3-Dienes. Control of 1,2- and 1,4-Addition Pathways and the Synthesis of Aza- and Oxa-bicyclic Systems" JOURNAL OF THE CHEMICAL SOCIETY, PERKIN TRANSACTIONS 1, 1977, Seiten 874-884, XP002104986 LETCHWORTH GB

## Beschreibung

Die vorliegende Erfindung betrifft ein neues Verfahren zur Herstellung von (1R,4S)- oder (1S,4R)-1-Amino-4-(hydroxymethyl)-2-cyclopenten der Formeln 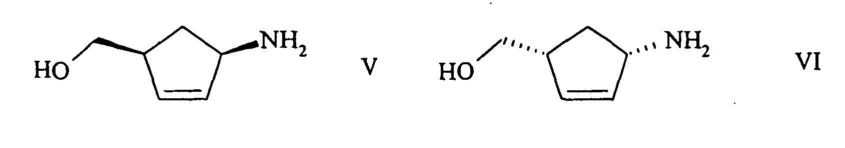 und deren Salzen, sowie deren acylierten Derivaten.

(1R,4S)-1-Amino-4-(hydroxymethyl)-2-cyclopenten ist ein wichtiges Zwischenprodukt zur Herstellung von carbocyclischen Nukleosiden wie z. B. Carbovir^{®} (Campbell et al., J. Org. Chem. 1995,60,4602 - 4616).

Ein Verfahren zur Herstellung von (1R,4S)-1-Amino-4-(hydroxymethyl)-2-cyclopenten ist beispielsweise von Campbell et al. (ibid) und von Park K. H. & Rapoport H. (J. Org. Chem. 1994, 59, 394 - 399) beschrieben.
Bei diesem Verfahren dient als Edukt entweder D-glucon-δ-lacton oder D-Serin, wobei ca. 15 Synthesestufen bis zur Bildung von (1R,4S)-N-tert-Butoxycarbonyl-4-hydroxymethyl-2-cyclopenten, welches dann zum (1R,4S)-1-Amino-4-(hydroxymethyl)-2-cyclopenten deprotektioniert wird, notwendig sind.
Diese beiden Verfahren sind kostspielig, umständlich und grosstechnisch nicht gangbar.
Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid zum gewünschten Produkt reduziert wird.
Nachteilig bei diesem Verfahren ist zum einen, dass auch die Doppelbindung des Cyclopentenringes reduziert wird, die schlechte Handhabbarkeit von Lithiumaluminiumhydrid und zum anderen, dass es zu kostspielig ist.
Taylor S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (+)-2-Azabicyclo[2.2.1]hept-5-en-3-on als Edukt. Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert-butyldicarbonat in das (1R,4S)-N-tert-butoxycarbonyl-2-azabicyclo[2.2.1]hept-5-en-3-on umgesetzt wird. Letzteres wird mit Natriumborhydrid und Trifluoressigsäure zum gewünschten Produkt reduziert. Auch ist dieses Verfahren viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.
Bekannt ist auch, dass man N-substituierte-(±)-2-Azabicyclo[2.2.1]hept-5-en-3-one, welche einen elektronenziehenden Substituenten tragen, mit einem Metallhydrid zu den entsprechenden N-substituierten Aminoalkoholen reduzieren kann (Katagiri et al., Tetrahedron Letters, 1989, 30, 1645 - 1648; Taylor et al., ibid).
Im Gegensatz dazu ist bekannt, dass unsubstituiertes (±)-2-Azabicyclo[2.2.1]hept-5-en-3-on der Formel 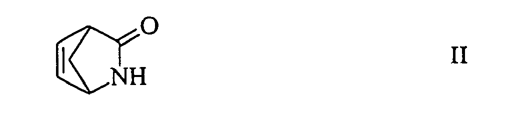 mit Lithiumaluminiumhydrid zu (±)-2-Azabicyclo[2.2.1]octen reduziert wird (Malpass & Tweedle, J. Chem. Soc., Perkin Trans 1, 1977, 874 - 884) und dass die direkte Reduktion von (±)-2-Azabicyclo[2.2.2]hept-5-en-3-on zum entsprechenden Aminoalkohol bislang als nicht möglich galt (Katagiri et al., ibid; Taylor et al., ibid).

Bekannt ist auch, racemisches 1-Amino-4-(hydroxymethyl)-2-cyclopenten mittels (-)- Dibenzoylweinsäure zu spalten (US-A 5 034 394 , EP434450). Diese Umsetzung hat einerseits den Nachteil, dass die (-)-Dibenzoylweinsäure teuer ist, anderseits, dass die Trennung in Gegenwart eines genau definierten Gemisches von Acetonitril und Ethanol erfolgen muss. Dieses Lösungsmittelgemisch kann nicht getrennt werden und muss der Verbrennung zugeführt werden.

Aufgabe der vorliegenden Erfindung war es, ein einfaches, ökonomisches und kostengünstiges Verfahren zur Herstellung von (1R,4S)-1-Amino-4-(hydroxymethyl)-2-cyclopenten zur Verfügung zu stellen.

Diese Aufgabe wurde entsprechend Anspruch 1 gelöst, indem der racemische, bevorzugt der cis-racemische, Aminoalkohol der Formel I mit einer biotechnologischen Racematspaltung mittels einer Hydrolase in Gegenwart eines Acylierungsmittels in die (1S,4R)- oder (1R,4S)-1-Amino-4-(hydroxymethyl)-2-cyclopentene der Formel 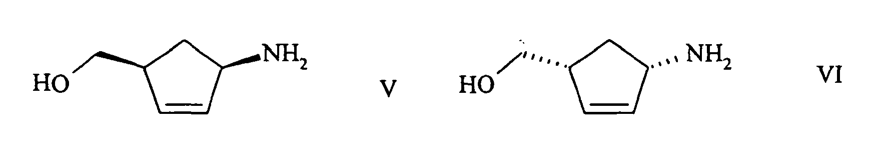 bzw. deren Salze und/oder in die (1S,4R)- oder (1R,4S)-1-Amino-4-(hydroxymethyl)-2-cyclopentenderivate der allgemeinen Formeln 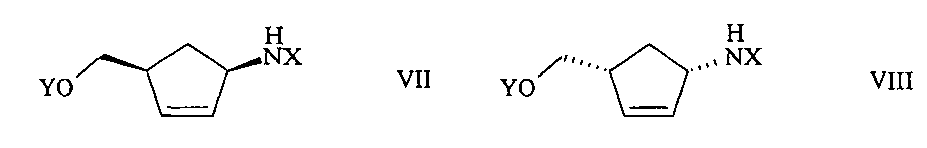 bzw. deren Salze, worin X und Y gleich oder verschieden sind und eine Acylgruppe oder H bedeuten, ausgenommen X = Y = H, umgesetzt wird.

Unter Salzen werden im folgenden Hydrohalogenidsalze wie Hydrochloride, Hydrobromide oder Tartrate verstanden.

Als Hydrolasen werden Lipasen, Proteasen, Amidasen oder Esterasen eingesetzt, zweckmässig werden Lipasen eingesetzt.

Wie fachmännisch bekannt, können hydrolasekatalysierte Acylierungen, bei denen optisch aktive Verbindungen gebildet werden, in Gegenwart eines geeigneten Acylierungsmittels durchgeführt werden (Balkenhohl et al., 1997, J. Prakt. Chem. 339, 381 - 384; K. Faber, "Biotransformation in Organic Chemistry", 2nd ed., Berlin 1995, 270 - 305).

Erfindungsgemäß werden als Acylierungsmittel Carbonsäureamide, Carbonsäureanhydride oder Carbonsäureester verwendet. Als Carbonsäureester können beispielsweise Alkoxycarbonsäureester wie Methoxyessigsäureethylester und Methoxyessigsäureisopropylester, C₁₋₆-Carbonsäureester wie Essigsäurebutylester, Buttersäureethylester und Hexansäureethylester, Glycerinester wie Tributyrin (Glycerintributyrat), Glykolester wie Glykoldibutyrat und Diglykolsäurediethylester, Dicarbonsäureester wie Fumar- und Malonsäurediethylester, Cyancarbonsäureester wie Cyanessigsäureethylester oder cyclische Ester wie beispielsweise 6-Caprolacton verwendet werden. Demzufolge entspricht die Acylgruppe in den Formeln VII und VIII der Säurekomponente des eingesetzten Carbonsäurederivats.

Als Lipase können handelsübliche Lipasen verwendet werden wie beispielsweise:
Novo-Lipase SP523 aus Aspergillus oryzae (Novozym 398), Novo-Lipase SP524 aus Aspergillus oryzae (Lipase = Palatase 20000L von Novo), Novo-Lipase SP525 aus Candida antarctica (Lipase B Novozym 435, immobilisiert), Novo-Lipase SP526 aus Candida antarctica (Lipase A = Novozym 735, immobilisiert), Lipase-kits von Fluka (1 & 2), Amano P Lipase, Lipase aus Pseudomonas sp., Lipase aus Candida cylindracea, Lipase aus Candida lipolytica, Lipase aus Mucor miehei, Lipase aus Aspergillus niger, Lipase aus Bacillus thermocatenulatus, Lipase aus Candida antarctica, Lipase AH (Amano; immobilisiert), Lipase P (Nagase), Lipase AY aus Candida rugosa, Lipase G (Amano 50), Lipase F (Amano F-AP15), Lipase PS (Amano), Lipase AH (Amano), Lipase D (Amano), Lipase AK aus Pseudomonas fluorescens, Lipase PS aus Pseudomonas cepacia, Newlase I aus Rhizopus niveus, Lipase PS-CI (immobilisierte Lipase aus Pseudomonas cepacia).
Diese Lipasen können, wie fachmännisch bekannt, als zellfreie Enzymextrakte oder auch in der entsprechenden Mikroorganismenzelle angewendet werden.

Als Proteasen können ebenfalls handelsübliche verwendet werden wie beispielsweise Serinproteasen wie Subtilisine. Als Subtilisin kann Savinase aus Bacillus sp., Alcalase, Subtilisin aus Bacillus licheniformis sowie Proteasen aus Aspergillus, Rhizopus, Streptomyces oder Bacillus sp. verwendet werden.

Zweckmässig wird die biotechnologische Racematspaltung bei einer Temperatur von 10 bis 80 °C und bei einem pH von 4 bis 9 durchgeführt.

Die biotechnologische Racematspaltung wird zweckmässig in einem protischen oder aprotischen organischen Lösungsmittel durchgeführt. Als aprotische organische Lösungsmittel sind Ether wie tert-Butylmethylether, Diisopropylether, Dibutylether, Dioxan und Tetrahydrofuran, aliphatische Kohlenwasserstoffe wie Hexan, organische Basen wie Pyridine und Carbonsäureester wie Ethylacetat und als protische organische Lösungsmittel die bereits beschriebenen C₁₋₆-Alkohole wie z. B. Pentanol geeignet.

Die bei der biotechnologischen Racematspaltung erfindungsgemäss gebildeten (1S,4R)- oder (1R,4S)-1-Amino-4-(hydroxymethyl)-2-cyclopentenderivate der allgemeinen Formeln VII und VIII werden, abhängig von der gewünschten Zielverbindung (Aminoalkohol der Formel V oder VI), auf chemische Weise zum Aminoalkohol der Formel V oder VI hydrolysiert. Die chemische Hydrolyse erfolgt zweckmässig in einer wässrigen basischen Lösung oder mittels eines basischen Ionenaustauschers. Als wässrige basische Lösung wird vorzugsweise ein Alkalimetallhydroxid eingesetzt. Als Alkalimetallhydroxid kann Natrium- oder Kaliumhydroxid eingesetzt werden. Als basische Ionenaustauscher können beispielsweise Dowex 1x8(OH⁻) und Duolite A147 eingesetzt werden.

Ein weiterer Gegenstand der vorliegenden Erfindung ist die Herstellung der (1R,4S)- oder (1S,4R)-1-Amino-4-(hydroxymethyl)-2-cyclopentenderivate der allgemeinen Formeln 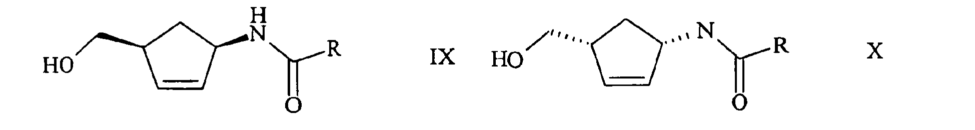 worin R C₁₋₄-Alkyl, C₁₋₄-Alkoxy, Aryl oder Aryloxy bedeutet, durch Weiterumsetzung, Acylierung, der (1R,4S)- oder (1S,4R)-1-Amino-4-(hydroxymethyl)-2-cyclopentene der Formeln V und VI.

C₁₋₄-Alkyl kann substituiert oder unsubstituiert sein. Unter substituiertem C₁₋₄-Alkyl wird im folgenden mit einem oder mehreren Halogenatomen substituiertes C₁₋₄-Alkyl verstanden. Als Halogenatom kann F, Cl, Br oder J angewendet werden. Beispiele für C₁₋₄-Alkyl sind Methyl, Ethyl, Propyl, Butyl, Isobutyl, tert. Butyl, Isopropyl, Chlormethyl, Brommethyl, Dichlormethyl, Dibrommethyl. Vorzugsweise wird als C₁₋₄-Alkyl Methyl, Ethyl, Propyl, Butyl, Isobutyl oder Chlormethyl verwendet.

Als C₁₋₄-Alkoxy kann beispielsweise Methoxy, Ethoxy, Propoxy oder Butoxy verwendet werden. Als Aryl kann beispielsweise Phenyl oder Benzyl, substituiert oder unsubstituiert, verwendet werden. Als substituiertes Phenyl oder Benzyl wird im folgenden mit einem oder mehreren Halogenatomen substituiertes Phenyl oder Benzyl verstanden, wie bspw. Chlorbenzyl, Dichlorbenzyl, Bromphenyl oder Dibromphenyl.

Als Aryloxy kann beispielsweise Benzyloxy oder Phenoxy, substituiert mit den zuvor beschriebenen Substituenten oder unsubstituiert, verwendet werden.

Zur Herstellung der (1R,4S)- oder (1S,4R)-1-Amino-4-(hydroxymethyl)-2-cyclopentenderivate der allgemeinen Formeln IX und X wird (±)-2-Azabicyclo[2.2.1]hept-5-en-3-on der Formel 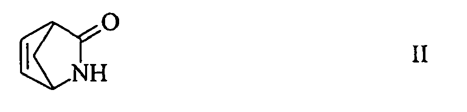 in Form des Racemats oder eines seiner optisch aktiven Isomere, mit einem Metallhydrid zum Aminoalkohol der Formel 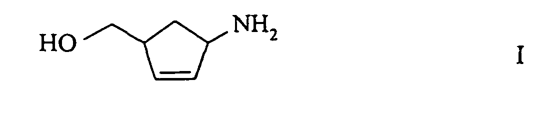 in Form des Racemats oder eines seiner optisch aktiven Isomere, reduziert.

Vorzugsweise wird der racemische cis-Aminoalkohol der Formel I erhalten.

Wie fachmännisch üblich kann der Aminoalkohol der Formel I mit einer Säure in die entsprechenden Salze überführt werden, wie beispielsweise in Hydrohalogenidsalze. Als Hydrohalogenidsalze sind Hydrobromide und Hydrochloride geeignet.

Das Edukt, das (±)-2-Azabicyclo[2.2.1]hept-5-en-3-on, kann gemäss der EP-A 0 508 352 hergestellt werden.

Als Metallhydride können Alkalimetall- oder Erdalkalimetallhydride sowie binäre oder komplexe Metallhydride der Bor- oder Aluminiumgruppe eingesetzt werden, beispielsweise Alkalimetall- und Erdalkalimetallborhydride und Alkalimetall- und Erdalkalimetallaluminiumhydride.

Als Alkalimetall- oder Erdalkalimetallhydride sind LiH, NaH, KH, BeH₂, MgH₂ oder CaH₂ geeignet. Als binäre Alkalimetall- oder Erdalkalimetallborhydride können NaBH₄, LiBH₄, KBH₄, NaAlH₄, LiAlH₄, KAlH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M² HₙLₘ haben, worin n eine ganze Zahl von 1 bis 4 ist und m eine ganze Zahl von 4 bis 4 minus der entsprechenden Zahl n ist, M¹ ein Alkalimetallatom bedeutet, M² Bor oder Aluminium bedeutet und L C₁₋₄-Alkyl, C₁₋₄-Alkenyl, C₁₋₄-Alkoxy, CN oder ein Amin ist oder die komplexen Metallhydride können die allgemeine Formel M²HₒLₚ haben, worin M² die genannte Bedeutung hat und O eine ganze Zahl von 0 bis 3 ist und p eine ganze Zahl von 3 bis 3 minus der entsprechenden Zahl p ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃)₃, NaAlH₂(OC₂H₄OCH₃)₂, NaAlH₂(C₂H₅)₂ oder NaBH₃CN eingesetzt werden. Vorzugsweise wird die Reduktion mit einem Metallborhydrid durchgeführt. Wie fachmännisch bekannt, können die erwähnten Metallhydride wie z. B. LiBH₄ auch "in situ" erzeugt werden. Gängige Darstellungsmethoden für LiBH₄ sind beispielsweise die Umsetzung eines Alkalimetallborhydrids mit einem Lithiumhalogenid (H. C. Brown et al., Inorg. Chem. 20, 1981, 4456 - 4457), die Umsetzung von LiH mit B₂O₃ in Gegenwart von Wasserstoff und einem Hydrierkatalysator (EP-A 0 512 895), die Umsetzung von LiH mit (H₅C₂)OBF₃ (DE-OS 94 77 02) und die von LiH mit B(OCH₃)₃ (US-A 2,534,533).

Zweckmässig werden die Metallhydride in einem molaren Verhältnis von 1 bis 5 pro mol (±)-2-Azabicyclo[2.2. 1]hept-5-en-3-on eingesetzt.

Vorzugsweise werden die Metallhydride, insbesondere NaBH₄, mit Lithiumsalzzusätzen verwendet. Als Lithiumsalze können LiCl, LiBr, LiF, LiJ, Li₂SO₄, LiHSO₄, Li₂CO₃, Li(OCH₃) und LiCO₃ verwendet werden.

Zweckmässig wird die Reduktion unter Inertgasatmosphäre, beispielsweise unter Argon- oder Stickstoffatmosphäre, durchgeführt.

Die Reduktion kann bei einer Temperatur von -20 bis 200 °C, vorzugsweise bei einer Temperatur von 60 bis 150 °C, durchgeführt werden.

Als Lösungsmittel sind aprotische oder protische organische Lösungsmittel geeignet. Als aprotische organische Lösungsmittel können Ether oder Glykolether wie beispielsweise Diethylether, Dibutylether, Ethylmethylether, Diisopropylether, tert-Butylmethylether, Anisol, Dioxan, Tetrahydrofuran, Mono-Glyme, Diglyme und Formaldehyddimethylacetal eingesetzt werden. Als protische organische Lösungsmittel sind C₁₋₆-Alkohole wie Methanol, Ethanol, Propanol, Isopropanol, Butanol, tert-Butanol, Pentanol, tert-Amylalkohol oder Hexanol sowie Mischungen von diesen mit Wasser geeignet. Als protische organische Lösungsmittel ebenso geeignet sind Mischungen von einem der genannten Ether, Glykolether mit Wasser oder mit einem der genannten Alkohole wie eine Mischung von einem C₁₋₆-Alkohol mit einem Ether oder Glykolether, insbesondere eine Mischung von Methanol, Ethanol oder Wasser mit Diethylether, Tetrahydrofuran, Dioxan, Glyme oder Diglyme. Vorzugsweise wird als Lösungsmittel ein protisches organisches Lösungsmittel, wie eine Mischung von einem C₁₋₆-Alkohol oder Wasser mit einem Ether oder Glykolether, eingesetzt.

In einer bevorzugten Ausführungsform wird die Reduktion in Gegenwart eines Zusatzes, beispielsweise in Gegenwart von Wasser oder eines ein- oder mehrwertigen C₁₋₆-Alkohols durchgeführt. Als einwertige C₁₋₆-Alkohol können Methanol, Ethanol, Methoxyethanol, n-Propanol, Isopropanol, Isobutanol, tert-Butanol, n-Butanol verwendet werden. Als mehrwertige Alkohole können bspw. Diole wie Butandiol und Triole wie Glycerin verwendet werden. Insbesondere wird als C₁₋₆-Alkohol Methanol oder Ethanol verwendet. Zweckmässig wird dabei der C₁₋₆-Alkohol in einem molaren Verhältnis von 2 bis 15 pro mol (±)-2-Azabicyclo[2.2.1]hept-5-en-3-on eingesetzt.

Wird die Umsetzung in Gegenwart der genannten Alkohole durchgeführt, kann in situ (intermediär) der entsprechende Aminosäureester gebildet werden. D. h. wird als Edukt (+)-2-Azabicyclo[2.2.1]hept-5-en-3-on verwendet, kann erfindungsgemäss der entsprechende (+)-Aminosäureester gebildet werden.

Wird als Edukt (-)-2-Azabicyclo[2.2.1]hept-5-en-3-on verwendet, kann erfindungsgemäss dementsprechend der (-)-Aminosäureester intermediär gebildet werden.

Der auf diese Weise erhaltene Aminoalkohol der Formel I wird wie oben beschrieben mit einer Hydrolase in Gegenwart eines Acylierungsmittels in das (1S,4R)- oder (1R,4S)-1-Amino-4-(hydroxymethyl)-2-cyclopentene der Formel V oder VI überführt, die dann zu den Produkten der Formeln IX oder X acyliert werden.

Die Acylierung kann mit einem Carbonsäurehalogenid der allgemeinen Formel 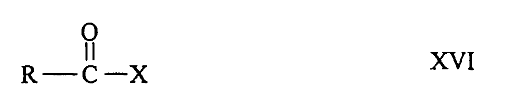 worin X ein Halogenatom bedeutet und R die genannte Bedeutung hat, oder mit einem Carbonsäureanhydrid der allgemeinen Formel 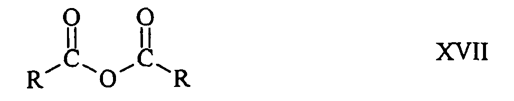 worin R die genannte Bedeutung hat, durchgeführt werden.

Als Halogenatom X können F, Cl, Br oder I verwendet werden. Vorzugsweise wird Cl oder F verwendet.

Beispiele für Carbonsäurehalogenide sind: Acetylchlorid, Chloracetylchlorid, Buttersäurechlorid, Isobuttersäurechlorid, Phenylessigsäurechlorid, Chlorameisensäurebenzylester (Cbz-Cl), Propionsäurechlorid, Benzoylchlorid, Chlorameisensäurealkylester oder tert-Butyloxycarbonylfluorid.

Beispiele für Carbonsäureanhydride sind: tert-Butoxycarbonylanhydrid, Buttersäureanhydrid, Essigsäureanhydrid oder Propionsäureanhydrid. Vorzugsweise wird die Acylierung mit einem Carbonsäureanhydrid, insbesondere mit tert-Butoxycarbonylanhydrid durchgeführt.
Die Acylierung kann ohne Lösungsmittel oder mit einem aprotischen organischen Lösungsmittel durchgerührt werden. Zweckmässig wird die Acylierung in einem aprotischen organischen Lösungsmittel durchgeführt. Als aprotisches organisches Lösungsmittel sind beispielsweise Pyridin, Acetonitril, Dimethylformamid, Diisopropylether, Tetrahydrofuran, Toluol, Methylenchlorid, N-Methylpyrrolidon, Triethylamin, Chloroform, Essigsäureethylester und Essigsäureanhydrid bzw. Mischungen von diesen geeignet.

Zweckmässig wird die Acylierung bei einer Temperatur von -20 bis 100 °C, vorzugsweise von 0 bis 80 °C, durchgeführt.

### Beispiele:

### Beispiel 1

### Reduktion von 2-Azabicyclo[2.2.1]hept-5-en-3-on

### 1.1 Herstellung von cis-(±)-1-Amino-4-(hydroacymethyl)-2-cyclopenten

Eine Suspension von (+)-2-Azabicyclo[2.2.1]hept-5-en-3-on (10,00 g, 91,6 mmol) und Lithiumborhydrid (4,00 g, 183,7 mmol) in trockenem Dioxan (100 ml) wurde unter Inertgasatmosphäre (Argon) 4 h bei 110 °C unter Rückflusstemperatur erhitzt. Nach dieser Zeit waren ca. 20 - 25 % des Eduktes zum Produkt umgesetzt (GC-Analyse mit internem Standard Benzophenon nach Aufarbeitung des Reaktionsgemisches; Aufarbeitung: 0,05 ml des Reaktionsgemisches wurden mit 0,1 ml 1 M HCl gequencht und direkt im Anschluss mit 0,2 ml 1M NaOH basisch gestellt). Der Strukturnachweis des Produktes erfolgte durch H-NMR, GC und GC-MS.

### 1.2 Herstellung von cis-(+)-1-Amino-4-(hydroxymethyl)-2-cyclopenten

In einem 25 ml Rundkolben wurden 1;0 g (9,2 mmol) (+)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepuffert. GC Analyse zeigte hier die Bildung des Produktes. Das gesamte Reaktionsgemisch wurde nun zur Trockene eingedampft und mittels Säulenchromatographie (Gradient: Hexan/Essigester/MeOH = 1:1:1-> MeOH) gereinigt. Auf diese Weise wurde cis-(+)-2-Azabicyclo[2.2.1]hept-5-en-3-on reisoliert und der entsprechende (+)-Aminoalkohol gewonnen.

### 1.3 Herstellung von cis-(-)-1-Amino-4-(hydroxymethyl)-2-cyclopenten

In einem 25 ml Rundkolben wurden 1,0 g (9,2 mmol) (-)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepufferet. GC Analyse zeigte hier die Bildung des Produktes in 18 % Ausbeute an. (GC-Standard ist Benzopherion). Das gesamte Reaktionsgemisch wurde nun zur Trockene eingedampft und mittels Säulenchromatographie (Gradient: Hexan/Essigester/MeOH = 1:1:1 -> MeOH) gereinigt. Auf diese Weise wurde 0,43g (43 %) cis-(-)-2-Azabicyclo[2.2.1]hept-5-en-3-on reisoliert sowie 0,04g (4 %) entsprechender (-)-Aminoalkohol gewonnen.
Durch HPLC war nur das (-)-Enantiomer des Aminoalkohols detektierbar. Der ee des Produktes ist somit >98 %.

### 1.4 Herstellung von cis-(±)-1-Amino-4-(hydroxymethyl)-2-cyclopenten in einem Alkohol

In einem 100 ml Rundkolben mit magnetischer Rührung wurden 3,0 g (27,5 mmol) (±)-2-Azabicyclo[2.2.1]hept-5-en-3-on und 1,2 g (28,3 mmol) Lithiumborhydrid, unter Inertgasatmosphäre, in 35 g 2-Butanol vorgelegt und 3 h bei 60 °C gerührt. GC-Analyse eines Musters (Aufarbeitung: 0,1 g Probe mit 0,2 ml 1M HCl sauergestellt, dann mit 0,1 ml gesättigter NaHCO₃ rasch basisch gestellt) zeigte nach dieser Zeit die Bildung des Produktes in 12 %iger Ausbeute an (GC-Standard ist Benzophenon).

### 1.5 Herstellung von cis-(±)-1-Amino-4-(hydroxymethyl)-2-cyclopenten in einem Alkohot/Ether-Gemisch

In einem 10 ml Rundkolben wurden unter Inertgasatmosphäre 0,5 g (4,6 mmol) (±)-2-Azabicyclo[2.2.1]hept-5-en-3-on und 0,59 g (18,4 mmol) Methanol in 7,5 ml Dioxan (abs.) vorgelegt. Dazu gab man 0,21 g (9,2 mmol) Lithiumborhydrid und heizte für 4 h bei 60°C. Dann kühlte man mit einem Eis/Wasser Bad auf 5°C und gab vorsichtig ca. 10 ml halbkonzentrierte HCl zum Reaktionsgemisch (heftige Reaktion, Gasentwicklung), wodurch eine gelblich klare Lösung entstand. Diese Lösung wurde direkt durch ein quantitatives ionenchromatografisches Verfahren analysiert. Sie enthielt 0,60 mmol (13,1%) cis-(±)-2-Azabicyclo[2.2.1]hept-5-en-3-on (bestimmt als HCl-Salz der entsprechenden Aminosäure, welche das saure Hydrolyseprodukt des (±)-2-Azabicyclo[2.2.1]hept-5-en-3-ons ist) und 3,06 mmol Produkt, entsprechend einer Ausbeute von 66,8%, Aminoalkohol.

### 1.6 Herstellung von cis-(±)-1-Amino-4-(hydroxymethyl)-2-cyclopenten in Gegenwart von Zusätzen wie Wasser oder verschiedenen Alkoholen

In einem 10 ml Rundkolben wurden 0,50 g (4,6 mmol) (±)-2-Azabicyclo[2.2.1]hept-5-en-3-on und 0,30 g (13,7 mmol) Lithiumborhydrid in 7,5 ml abs. Dioxan vorgelegt und auf 60 °C aufgeheizt. Bei dieser Temperatur wurde während 30 min. mittels Spritze X mmol des Zusatzes Y (Alkohol oder Wasser) zugetropft. Nun wurde 2 h bei 60 °C gerührt, abgekühlt auf ca. 20 °C und auf ca. 10 ml halbkonzentrierte HCl gegeben. Der Gehalt wurde dann direkt durch ein quantitatives ionenchromatografisches Verfahren bestimmt (vgl. Tabelle 1).

**Tabelle 1**

| Beispiel | Zusatz Y | X | X | (±)-2-Azabicyclo[2.2.1]-hept-5-en-3-on | Aminoalkohol |
|---|---|---|---|---|---|
| | | mmol | Aequivalente | Ausbeute % | |
| 1.6.1 | - | - | - | 15 | 52 |
| 1.6.2 | Wasser | 17,1 | 1,25 | 23,3 | 67,5 |
| 1.6.3 | Wasser | 34,3 | 2,5 | 32,3 | 58,3 |
| 1.6.4 | Methanol | 34,3 | 2,5 | 4,5 | 83,1 |
| 1.6.5 | Ethanol | 34,3 | 2,5 | 6,5 | 74,7 |
| 1.6.6. | Isopropanol | 34,3 | 2,5 | 28,1 | 52,3 |

### 1.7. Herstellung von cis-(±)-1-Amino-4-(hydroxymethyl)-2-cyclopenten mit verschiedenen Methanol-Mengen

Die Reaktion wurde unter den gleichen Bedingungen wie Beispiel 1.6 durchgeführt, jedoch mit der Ausnahme, dass anstatt des Zusatzes Y die Reaktion in verschiedenen Methanol-Konzentrationen durchgeführt wurde. Die Ergebnisse sind in Tabelle 2 zusammengefasst.

**Tabelle 2**

| Beispiel | Methanol | Methanol | (±)-2-Azabicyclo-[2.2.1] hept-5-en-3-on | Aminoalkohol |
|---|---|---|---|---|
| | mmol | Aequivalente | Ausbeute % | |
| 1.7.1 | 9,2 | 1 | 27,5 | 44,8 |
| 1.7.2 | 18,3 | 2 | 13,1 | 66,8 |
| 1.7.3 | 27,5 | 3 | 24,7 | 54,8 |
| 1.7.4 | 36,6 | 4 | 5,7 | 56,8 |
| 1.7.5 | 45,8 | 5 | 12,0 | 58,3 |
| 1.7.6 | 55,0 | 6 | 7,2 | 33,0 |

### 1.8 Herstellung von cis-(±)-1-Amino-4-(hydroxymethyl)-2-cyclopenten mit verschiedenen Lösungsmitteln

Die Reaktion wurde unter den gleichen Bedingungen wie Beispiel 1.6 durchgeführt, jedoch mit der Ausnahme, dass anstatt des Zusatzes Y 1,1 g Methanol zugetropft wurden und anstatt Dioxan als Lösungsmittel die Reaktion in verschiedenen Lösungsmitteln (7,5 ml) durchgeführt wurde und der Gehalt bestimmt wurde. Die Ergebnisse sind in Tabelle 3 zusammengefasst.

**Tabelle 3**

| Beispiel | Lösungsmittel X | (±)-2-Azabicyclo[2.2.1] hept-5-en-3-on | Aminoalkohol |
|---|---|---|---|
| | | Ausbeute % | |
| 1.8.1 | Dioxan | 13,6 | 79,8 |
| 1.8.2 | Diethylether | 10,8 | 68,6 |
| 1.8.3 | Tetrahydrofuran | 22,4 | 67,6 |
| 1.8.4 | Diisopropyl-Ether | 12,6 | 51,3 |
| 1.8.5 | tert-Butylmethyl-Ether | 10,0 | 71,3 |
| 1.8.6 | Mono-Glyme | 15,5 | 75,3 |
| 1.8.7 | Formaldehyddimethyl-acetal | 12,0 | 74,2 |

### 1.9 Herstellung von cis-(±)-1-Amino-4-(hydroxymethyl)-2-cyclopenten mit verschiedenen LiBH₄-Zugaben

Die Reaktion wurde unter den gleichen Bedingungen wie in Beispiel 1.6 durchgeführt, jedoch mit der Ausnahme, dass anstatt Zusatz Y 2,5 mol Methanol eingesetzt wurden und die Reaktion mit verschiedenen LiBH₄-Konzentrationen durchgeführt und der Gehalt bestimmt wurde. Die Ergebnisse sind in Tabelle 4 zusammengefasst.

**Tabelle 4**

| Beispiel | LiBH₄ | LiBH₄ | (±)-2-Azabicyclo[2.2.1] hept-5-en-3-on | Aminoalkohol |
|---|---|---|---|---|
| | mmol | Aequivalente | Ausbeute % | |
| 1.9.1 | 4,6 | 1 | 11,9 | 47,9 |
| 1.9.2 | 6,9 | 1,5 | 9,6 | 45,6 |
| 1.9.3 | 9,2 | 2 | 12,7 | 71,3 |
| 1.9.4 | 11,5 | 2,5 | 13,3 | 74,5 |
| 1.9.5 | 13,8 | 3 | 12,8 | 77,1 |
| 1.9.6 | 16,1 | 3,5 | 12,7 | 62,4 |

### 1.10 Herstellung von cis-(+)- oder (-)-1-Amino-4-(hydroxymethyl)-2-cyclopenten in Gegenwart verschiedener Akohole bzw. in Gegenwart von Wasser in verschiedenen Lösungsmitteln

In einem 10 ml Rundkolben mit magnetischer Rührung wurden 0,50 g (4,6 mmol) (+)-oder (-)-2-Azabicyclo[2.2.1]hept-5-en-3-on und 0,30 g (13,7 mmol) Lithiumborhydrid in 6 ml verschiedener Lösungsmittel vorgelegt und auf 60°C aufgeheizt. Bei dieser Temperatur wird während 30 min. mittels Spritze 34,3 mmol des Zusatzes Y zugetropft. Nun wurde 2h bei 60°C gerührt, abgekühlt auf ca. 20°C und auf ca. 10 ml halbkonzentrierte HCl gegeben.
Der Gehalt wird dann direkt durch ein quantitatives ionenchromatografisches Verfahren bestimmt (vgl. Tabelle 5). Der ee-Wert des Produktes wurde mittels HPLC ermittelt. Die Ergebnisse sind in Tabelle 5 zusammengefasst.

**Tabelle 5**

| | (-)-2-Azabicyclo[2.2.1] hept-5-en-3-on | (+)-2-Azabicyclo-[2.2.1]hept-5-en-3-on | | | Aminoalkohol | |
|---|---|---|---|---|---|---|
| Beispiel | ee-Wert | | Lösungsmittel | Zusatz Y | Ausbeute (IC) | ee-Wert (HPLC) |
| 1.10.1 | 98,0 | | Dioxan | Wasser | 64,3 | >99,0 |
| 1.10.2 | 98,0 | | Glyme | Wasser | 68,0 | >99,0 |
| 1.10.3 | 75,9 | | Dioxan | Wasser | 65,1 | 76,0 |
| 1.10.4 | 75,9 | | Glyme | Wasser | 63,5 | 75,6 |
| 1.10.5 | 50,2 | | Dioxan | Wasser | 74,8 | 51,4 |
| 1.10.6 | 51,6 | | Glyme | Wasser | 64,1 | 53,0 |
| 1.10.7 | 25,3 | | Dioxan | Wasser | 61,1 | 30,4 |
| 1.10.8 | 25,6 | | Glyme | Wasser | 61,0 | 29,6 |
| 1.10.9 | 98,0 | | Dioxan | Methanol | 83,1 | 98,2 |
| 1.10.10 | 98,0 | | Glyme | Methanol | 81,5 | 99,2 |
| 1.10.11 | 75,9 | | Dioxan | Methanol | 81,4 | 78,0 |
| 1.10.12 | 76,2 | | Glyme | Methanol | 79,9 | 78,6 |
| 1.10.13 | 50,4 | | Dioxan | Methanol | 81,3 | 54,4 |
| 1.10.14 | 51,5 | | Glyme | Methanol | 82,0 | 55,2 |
| 1.10.15 | 24,8 | | Dioxan | Methanol | 65,2 | 27,4 |
| 1.10.16 | 27,8 | | Glyme | Methanol | 81,7 | 32,2 |
| 1.10.17 | 98,0 | | Dioxan | Ethanol | 80,8 | 80,8 |
| 1.10.18 | 98,0 | | Glyme | Ethanol | 85,1 | 85,1 |
| 1.10.19 | 75,5 | | Dioxan | Ethanol | 85,3 | 78,2 |
| 1.10.20 | 75,6 | | Glyme | Ethanol | 83,6 | 78,4 |
| 1.10.21 | 50,7 | | Dioxan | Ethanol | 76,3 | 54,4 |
| 1.10.22 | 51,1 | | Glyme | Ethanol | 71,3 | 55,2 |
| 1.10.23 | 25,4 | | Dioxan | Ethanol | 73,0 | 28,6 |
| 1.10.24 | 25,5 | | Glyme | Ethanol | 75,0 | 28,6 |
| 1.10.25 | | 98,0 | Dioxan | Wasser | 62,0 | >99,0 |
| 1.10.26 | | 98,0 | Glyme | Wasser | 59,5 | >99,0 |
| 1.10.27 | | 51,3 | Dioxan | Wasser | 79,0 | 52,2 |
| 1.10.28 | | 49,0 | Glyme | Wasser | 61,3 | 52,0 |
| 1.10.29 | | 98,0 | Dioxan | Methanol | 77,2 | >99,0 |
| 1.10.30 | | 98,0 | Glyme | Methanol | 80,0 | >99,0 |
| 1.10.31 | | 49,0 | Dioxan | Methanol | 80,8 | 46,8 |
| 1.10.32 | | 49,5 | Glyme | Methanol | 80,9 | 48,8 |

### 1.11 Herstellung von cis-(±)-1-Amino-4-(hydroxymethyl)-2-cyclopenten mittels Natriumborhydrid in verschiedenen Alkoholen

Entsprechend zu Beispiel 1.6 wurde die Reaktion in verschiedenen Zusätzen (Alkohole oder Wasser) durchgeführt. Im Unterschied zu Beispiel 1.6 wurde jedoch Natriumborhydrid (0,51g; 13,7 mmol) als Reduktionsmittel eingesetzt. Die Ergebnisse sind in Tabelle 6 zusammengefasst.

**Tabelle 6**

| Beispiel | Zusatz Y | X | X | (±)-2-Azabicyclo[2.2.1] hept-5-en-3-on | Aminoalkohol |
|---|---|---|---|---|---|
| | | mmol | Aequivalente | Ausbeute % | |
| 1.11.1 | Wasser | 17,1 | 1,25 | 75,4 | 20,1 |
| 1.11.2 | Wasser | 34,3 | 2,5 | 71,9 | 26,7 |
| 1.11.3 | Methanol | 34,3 | 2,5 | 39,2 | 22,2 |
| 1.11.4 | Ethanol | 34,3 | 2,5 | 67,8 | 8,6 |
| 1.11.5 | - | - | - | 62,2 | 3,5 |

### 1.12 Herstellung von cis-(±)-1-Amino-4-(hydroxymethyl)-2-cyclopenten mit NaBH₃CN

In einem 100 ml Sulfierkolben mit mechanischer Rührung wurden 60 ml Dioxan und 8,6 g (137 mmol) Natriumcyanborhydrid sowie 11,9 g (137 mmol) Lithiumbromid über Nacht für 15 h bei 110°C rückflussiert. Dann wurde auf 60°C abgekühlt und eine Lösung von 5,0 g (45,8 mmol) (±)-2-Azabicyclo[2.2.1 ]hept-S-en-3-on mit 15 ml Methanol während 30 min. zugetropft. Die weisse Suspension wurde für 3 h bei 60°C gerührt, abgekühlt auf ca. 5°C und auf ca. 100 ml halbkonzentrierte HCl gegeben. Der Gehalt wurde dann direkt durch ein quantitatives ionenchromatografisches Verfahren bestimmt. Die Ausbeute an Aminoalkohol betrug ca. 4%.

### Beispiel 2

### Alkalische Hydrolyse von Acetyl-(±)-1-Amino-4-(hydroxymethyl)-2-cyclopenten

88,9 g racemisches Acetyl-1-amino-4-(hydroxymethyl)-2-cyclopenten (Gehalt 77,2 %) wurden suspendiert (teilweise gelöst) in 70 g Wasser. Dazu wurden 84 g 30 % NaOH (1,1 Aequivalente) zugegeben und die Lösung wurde 3 h rückflussiert. Laut DC war die Hydrolyse vollständig. Das entstandene Acetat wurde durch Elektrodialyse entfernt. Die erhaltene wässrige Lösung wurde eingeengt und getrocknet durch azeotrope Destillation mit Butanol. Der Rückstand wurde aufgenommen in Methanol zur Racematspaltung. Die Ausbeute der Hydrolyse zum (±)-1-Amino-4-(hydroxymethyl)-2-cyclopenten betrug 90 %.

### Beispiel 3: Herstellung von (1R,4S)- oder (1S,4R)-1-Amino-4-(hydroxymethyl)-2-cyclopenten

### 3.1 Racematspaltung mittels Hydrolasen

### 3.1.1 Herstellung von (1R,4S)-1-Amino-4-(hydroxymethyl)-2-cyclopenten mittels Lipasen

3.1.1.1 25 mM racemisches cis-1-Amino-4-(hydroxymethyl)-2-cyclopenten wurden mit 1000 Units Novozym 435 in 5 ml Dioxan bei Raumtemperatur suspendiert. 25 mM Methoxyessigsäureethylester wurden als Acetylierungsmittel zugesetzt. Die Bildung von N-Methoxyacetylaminoalkohol konnte mit DC eindeutig nachgewiesen werden. Der Umsatz betrug 50 % (nach Schätzung des DC). Dabei wurde das (1R,4S)-1-Amino-4-(hydroxymethyl)-2-cyclopenten gebildet.
3.1.1.2 50 mM racemisches cis-1-Amino-4-(hydroxymethyl)-2-cyclopenten wurden mit 1000 Units (U) Novozym 435 in 5 ml Tetrahydrofuran suspendiert. Dieses wurde mit 50 mM NaOH und 50 mM Methoxyessigsäureethylester versetzt und bei 30 °C inkubiert. N-Methoxyacetylaminoalkohol konnte mit DC nachgewiesen werden. Der geschätzte Umsatz betrug 50 %. Dabei wurde das (1R,4S)-1-Amino-4-(hydroxymethyl)-2-cyclopenten gebildet.
3.1.1.3 11 mg racemisches cis-1-Amino-4-(hydroxymethyl)-2-cyclopenten wurden mit 1 ml Methyl-tert.-butylether, 0,06 ml Tributyrin (Glycerintributyrat) und 20 U Novozym 435 (immob. Lipase aus Candida antarctica) bei Raumtemperatur gerührt. Nach 3 Tagen wurde laut HPLC enantiomerenreines (1R, 4S)-1-Amino-4-(hydroxymethyl)-2-cyclopenten in 43% Ausbeute erhalten.
3.1.1.4 11 mg racemisches cis-1-Amino-4-(hydroxymethyl)-2-cyclopenten wurden mit 1 ml Methyl-tert.-butylether, 0,02 ml 6-Caprolacton und 20 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 87% ee in 49% Ausbeute erhalten (HPLC).
3.1.1.5 100 mg racemisches cis-1-Amino-4-(hydroxymethyl)-2-cyclopenten wurden mit 1 ml Hexan, 0,3 ml Tributyrin und 20 U Novozym 435 (immob. Lipase aus Candida antarctica) bei Raumtemperatur gerührt. Nach 1 Woche wurde (1R, 4S)-1-Amino-4-(hydroxymethyl)-2-cyclopenten mit 77% ee in 28% Ausbeute erhalten (HPLC).
3.1.1.6 100 mg racemisches cis-1-Amino-4-(hydroxymethyl)-2-cyclopenten wurden mit 1 ml tert.-Butanol, 0,3 ml Tributyrin und 20 U Novozym 435 (immob. Lipase aus Candida antarctica) bei 30°C gerührt. Nach 1 Woche wurde (1R, 4S)-1-Amino-4-(hydroxymethyl)-2-cyclopenten mit 78% ee in 15% Ausbeute erhalten (HPLC).
3.1.1.7 11 mg racemisches cis-1-Amino-4-(hydroxymethyl)-2-cyclopenten wurden mit 1 ml Methyl-tert.-butylether, 0,2 mmol Capronsäuremethylester und 20 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 68% ee in 52% Ausbeute erhalten (HPLC).
3.1.1.8 11 mg racemisches eis-1-Amino-4-(hydroxymethyl)-2-cyclopenten wurden mit 1 ml Methyl-tert.-butylether, 0,2 mmol Glykol-di-butyrat und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 89% ee in 31 % Ausbeute erhalten (HPLC).
3.1.1.9 11 mg racemisches cis-1-Amino-4-(hydroxymethyl)-2-cyclopenten wurden mit 1 ml Methyl-tert.-butylether, 0,2 mmol Fumarsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 86% ee in 26% Ausbeute erhalten (HPLC).
3.1.1.10 11 mg racemisches cis-1-Amino-4-(hydroxymethyl)-2-cyclopenten wurden mit 1 ml Methyl-tert.-butylether, 0,2 mmol Malonsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 86% ee in 21% Ausbeute erhalten (HPLC).
3.1.1.11 11 mg racemisches cis-1-Amino-4-(hydroxymethyl)-2-cyclopenten wurden mit 1 ml Diisopropylether, 0,2 mmol Tributyrin und 40 U Novozym 435 (immob. Lipase aus Candida antarctica) bei Raumtemperatur gerührt. Nach 4 Tagen wurde enantiomerenreines (1R, 4S)-1-Amino-4-(hydroxymethyl)-2-cyclopenten in 15% Ausbeute erhalten (HPLC).
3.1.1.12 11 mg racemisches eis-1-Amino-4-(hydroxymethyl)-2-cyclopenten wurden mit 1 ml Diisopropylether, 0,2 mmol Fumarsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 88% ee in 24% Ausbeute erhalten (HPLC).
3.1.1.13 11 mg racemisches cis-1-Amino-4-(hydroxymethyl)-2-cyclopenten wurden mit 1 ml Diisopropylether, 0,2 mmol Malonsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 82% ee in 14% Ausbeute erhalten (HPLC).
3.1.1.14 11 mg racemisches cis-1-Amino-4-(hydroxymethyl)-2-cyclopenten wurden mit 1 ml Diisopropylether, 0,2 mmol Diglykolsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 88% ee in 7% Ausbeute erhalten (HPLC).
3.1.1.15 11 mg racemisches cis-1-Amino-4-(hydroxymethyl)-2-cyclopenten wurden mit 1 ml Dibutylether, 0,2 mmol Tributyrin und 40 U Novozym 435 (immobilisierte Lipase aus Candida antarctica) bei Raumtemperatur gerührt. Nach 4 Tagen wurde (1R, 4S)-1-Amino-4-(hydroxymethyl)-2-cyclopenten mit 95% ee in 13% Ausbeute erhalten (HPLC).
3.1.1.16 11 mg racemisches cis-1-Amino-4-(hydroxymethyl)-2-cyclopenten wurden mit 1 ml Pyridin, 0,02 ml 2-Methoxyessigsäureethylester und 20 mg Lipase AK (Lipase aus Pseudomonas fluorescens) bei Raumtemperatur gerührt. Nach 4 Tagen wurde (1R, 4S)-1-Amino-4-(hydroxymethyl)-2-cyclopenten mit 84% ee in 18% Ausbeute erhalten (HPLC).
3.1.1.17 11 mg racemisches cis-1-Amino-4-(hydroxymethyl)-2-cyclopenten wurden mit 1 ml Methyl-tert.-butylether, 0,2 mmol Cyanessigsäureethylester und 10 mg Lipase PS (Lipase aus Pseudomonas cepacia) bei Raumtemperatur gerührt. Nach 4 Tagen wurde (1R, 4S)-1-Amino-4-(hydroxymethyl)-2-cyclopenten mit 67% ee in 40% Ausbeute erhalten (HPLC).
3.1.1.18 11 mg racemisches cis-1-Amino-4-(hydroxymethyl)-2-cyclopenten wurden mit 1 ml Methyl-tert.-butylether, 0,2 mmol Fumarsäurediethylester und 10 mg Lipase PS (Lipase aus Pseudomonas cepacia) bei Raumtemperatur gerührt. Nach 4 Tagen wurde (1R, 4S)-1-Amino-4-(hydroxymethyl)-2-cyclopenten mit 86% ee in 18% Ausbeute erhalten (HPLC).

### 3.1.2 Herstellung von (1R,4S)-1-Amino-4-(hydroxymethyl)-2-cyclopenten mittels Proteasen

3.1.2.1 11 mg racemisches cis-1-Amino-4-(hydroxymethyl)-2-cyclopenten wurden mit 1 ml 2-Methyl-2-butanol, 0,2 mmol Maleinsäurediethylester und 40 mg Alcalase (Protease aus Bacillus licheniformis) bei Raumtemperatur gerührt. Nach 4 Tagen wurde (1R, 4S)-1-Amino-4-(hydroxymethyl)-2-cyclopenten mit 28% ee in 39% Ausbeute erhalten (HPLC).
3.1.2.2 11 mg racemisches cis-1-Amino-4-(hydroxymethyl)-2-cyclopenten wurden mit 1 ml 2-Methyl-2-butanol, 0,2 mmol Fumarsäurediethylester und 40 mg Savinase (Protease aus Bacillus sp.) bei Raumtemperatur gerührt. Nach 4 Tagen wurde (1R, 4S)-1-Amino-4-(hydroxymethyl)-2-cyclopenten mit 32% ee in 42% Ausbeute erhalten (HPLC).
3.1.2.3 11 mg racemisches cis-1-Amino-4-(hydroxymethyl)-2-cyclopenten wurden mit 1 ml 2-Methyl-2-butanol, 0,06 ml Tributyrin und 20 mg Savinase (Protease aus Bacillus sp.) bei Raumtemperatur gerührt. Nach 4 Tagen wurde (1R, 4S)-1-Amino-4-(hydroxymethyl)-2-cyclopenten mit 22% ee in 39% Ausbeute erhalten (HPLC).
3.1.2.4 11 mg racemisches cis-1-Amino-4-(hydroxymethyl)-2-cyclopenten wurden mit 1 ml 2-Methyl-2-butanol, 0,06 ml Tributyrin und 20 mg Subtilisin (Protease aus Bacillus licheniformis) bei Raumtemperatur gerührt. Nach 4 Tagen wurde (1 R, 4S)-1-Amino-4-(hydroxymethyl)-2-cyclopenten mit 23% ee in 36% Ausbeute erhalten (HPLC).

### 3.1.3 Herstellung von (1S,4R)-1-Amino-4-(hydroxymethyl)-2-cyclopenten mittels Proteasen

3.1.3.1 11 mg racemisches cis-1-Amino-4-(hydroxymethyl)-2-cyclopenten wurden mit 1 ml Hexan, 0,06 ml Tributyrin und 120 U Savinase (Protease aus Bacillus sp.) bei Raumtemperatur gerührt. Nach 3-6 Tagen wurde (1S, 4R)-1-Amino-4-(hydroxymethyl)-2-cyclopenten mit 44% ee in 46% Ausbeute erhalten (HPLC).
3.1.3.2 11 mg racemisches cis-1-Amino-4-(hydroxymethyl)-2-cyclopenten wurden mit 1 ml Hexan, 0,06 ml Tributyrin und 20 mg Alcalase (Protease aus Bacillus licheniformis) bei Raumtemperatur gerührt. Nach 3 - 6 Tagen wurde (1S, 4R)-1-Amino-4-(hydroxymethyl)-2-cyclopenten mit 44% ee in 35% Ausbeute erhalten (HPLC).

### 3.1.4 Herstellung von (1S,4R)-1-Amino-4-(hydroxymethyl)-2-cyclopenten mittels Lipasen

3.1.4.1 11 mg racemisches cis-1-Amino-4-(hydroxymethyl)-2-cyclopenten wurden mit 1 ml 2-Methyl-2-butanol, 0,03 ml Ethylbutyrat und 20 mg Newlase F (Lipase aus Rhizopus niveus) bei Raumtemperatur gerührt. Nach 1 Woche wurde (1S, 4R)-1-Amino-4-(hydroxymethyl)-2-cyclopenten mit 39% ee in 37% Ausbeute erhalten (HPLC).
3.1.4.2 11 mg racemisches cis-1-Amino-4-(hydroxymethyl)-2-cyclopenten wurden mit 1 ml Pyridin, 0,06 ml Tributyrin und 20 mg Lipase AK (Lipase aus Pseudomonas fluorescens) bei Raumtemperatur gerührt. Nach 1 Woche wurde (1S, 4R)-1-Amino-4-(hydroxymethyl)-2-cyclopenten mit 30% ee in 10% Ausbeute erhalten (HPLC).
3.1.4.3 11 mg racemisches cis-1-Amino-4-(hydroxymethyl)-2-cyclopenten wurden mit 1 ml 2-Methyl-2-butanol, 0,06 ml Tributyrin und 20 mg Lipase AY (Lipase aus Candida rugosa) bei Raumtemperatur gerührt. Nach 1 Woche wurde (1S, 4R)-1-Amino-4-(hydroxymethyl)-2-cyclopenten mit 32% ee in 13% Ausbeute erhalten (HPLC).
3.1.4.4 11 mg racemisches cis-1-Amino-4-(hydroxymethyl)-2-cyclopenten wurden mit 1 ml Methyl-t.-butylether, 0,06 ml Tributyrin und 20 mg Lipase PS-CI (immobilisierte Lipase aus Pseudomonas cepacia) bei Raumtemperatur gerührt. Nach 1 Woche wurde (1S, 4R)-1-Amino-4-(hydroxymethyl)-2-cyclopenten mit 29% ee in 16% Ausbeute erhalten (HPLC).
3.1.4.5 11 mg racemisches cis-1-Amino-4-(hydroxymethyl)-2-cyclopenten wurden mit 1 ml Methyl-t.-butylether, 0,06 ml Tributyrin und 20 mg Lipase PS (Lipase aus Pseudomonas cepacia) bei Raumtemperatur gerührt. Nach 1 Woche wurde (1S, 4R)-1-Amino-4-(hydroxymethyl)-2-cyclopenten mit 24% ee in 22% Ausbeute erhalten (HPLC).

### Beispiel 4: Herstellung von (1R,4S)-Amino-4-(hydroxymethyl)-2-cyclopentenhydrochlorid

### 4.1 Reduktion von (-)-2-Azabicyclo[2.2.1]hept-5-en-3-on

In einen mit N₂ inertisierten 2 1- Autoklaven (V4A) wurden 61,4 g Natriumborhydrid 97,5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130 °C aufgeheizt und 4,5 Stunden bei dieser Temperatur gerührt (max. 8,0 bar).

Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 11-Glasrührwerk durch Destillation bei
Normaldruck auf ca. die Hälfte eingeengt (Destillat 1: ca. 710 g Tetrahydrofuran).
Anschliessend wurde durch weitere Destillation, im Wechsel mit portionsweiser Zugabe von insgesamt 936 g Dioxan der Lösungsmitteltausch vollendet (Destillat 2: ca. 1289 g Tetrahydrofuran/Dioxan).
Zur auf ca. 60 °C gekühlten LiBH₄-Suspension wurden 56,7 g (-)-2-Azabicyclo[2.2.1]hept-5-en-3-on (97,5%) addiert.
Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Durch Destillation bei Normaldruck (bis zu einer Kopftemperatur von 75 °C) wurden die Leichtsieder (Methanol, Borester) und ca. 70% des Dioxans entfernt (Destillat 3: ca. 1093 g). Anschliessend wurde durch Vakuumdestillation (ca. 30 mbar), im Wechsel mit portionsweiser Zugabe von insgesamt 282 g 1-Pentanol der Lösungsmitteltausch vollendet (Destillat 4: ca. 240 g Dioxan / Pentanol). Nach Zugabe weiterer 302 g 1-Pentanol wurde 1 Stunde bei 50 °C gerührt und die ausgefallenen Salze, ca. 39 g Feuchtgewicht, wurden abfiltriert und mit 200 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 236 g Aceton zudosiert und die Reaktionsmischung wurde mit einigen Kristallen (1R,4S)-Amino-4-(hydroxymethyl)-2-cyclopenten angeimpft. Innerhalb einer Stunde wurde auf 5 °C abgekühlt und zur Vollendung der Kristallisation wurde weitere 6 h bei 5 °C gerührt.
Das Kristallisat wurde abfiltriert, mit 63 g Aceton gewaschen und bei max. 50 °C im Vakuumtrockenschrank (10 mbar) getrocknet. Man erhielt 83,5 g Rohprodukt * (Gehalt: 56,5%).
Dies entsprach einer Ausbeute von 61,4 % bzgl. eingesetztem (-)-2-Azabicyclo-[2.2.1]hept-5-en-3-on.

### 4.2 Reduktion von (±)-2-Azabicyclo[2.2.1]hept-5-en-3-on

In einen mit N₂ inertisierten 2 1- Autoklaven (V4A) wurden 41,56 g Natriumborhydrid 97,5% (1,071 Mol), 51,48 g Lithiumchlorid 98,5% (1,196 Mol), 9,30 g Celite sowie 955,0 g Tetrahydrofuran eingefüllt. Der Autoklav wurde verschlossen, es wurde auf eine Innentemperatur von 130 °C aufgeheizt und 6 Stunden bei dieser Temperatur gerührt (max. 6,3 bar).
Nach Abkühlen auf ca. 60 °C wurden die in Tetrahydrofuran unlöslichen Natriumsalze (NaCl, NaBH₄) abfiltriert. Diese wurden mit 239,0 g Tetrahydrofuran gewaschen und die vereinigten Filtrate wurden in einem 11-Glasrührwerk durch Destillation bei Normaldruck auf ca die Hälfte eingeengt (Destillat 1: ca. 590 g THF).
Anschliessend wurde durch weitere Destillation, im Wechsel mit portionsweiser Zugabe von insgesamt 661,0 g Dioxan der Lösungsmitteltausch vollendet (Destillat 2: ca. 685 g Tetrahydrofuran/Dioxan). Zur auf ca. 60 °C gekühlten LiBH₄-Suspension wurden 36,0 g 2-Azabicyclo[2.2.1]hept-5-en-3-on (97,5%) addiert. Bei ca. 60 °C beginnend wurden in exakt einer Stunde 77,6 g Methanol so zudosiert, dass ein Temperaturbereich von 58 - 62 °C eingehalten wurde. Es wurde eine weitere Stunde bei 60 °C nachreagieren gelassen. Anschliessend wurden weitere 233,0 g Methanol addiert und der Rührwerksinhalt wurde auf 0 °C abgekühlt. Bei dieser Temperatur wurden 52,9 g HCl in die Reaktionsmischung eingeleitet (leicht exotherm) und es wurde eine weitere Stunde bei ca 0 °C gerührt.
Durch Destillation bei Normaldruck (bis zu einer Kopftemperatur von 75 °C) wurden die Leichtsieder (Methanol, Borester) und ca. 70% des Dioxans entfernt (Destillat 3: ca. 700 g).

Anschliessend wurde durch Vakuumdestillation (ca. 30 mbar), im Wechsel mit portionsweiser Zugabe von insgesamt 169,4 g 1-Pentanol der Lösungsmitteltausch vollendet (Destillat 4: ca. 183 g Dioxan / Pentanol). Nach Zugabe weiterer 127,1 g 1-Pentanol wurde 1 Stunde bei 50 °C gerührt und die ausgefallenen Salze, ca.41 g Feuchtgewicht, wurden abfiltriert und mit 63,5 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 238,0 g Aceton zudosiert und die Reaktionsmischung wurde mit einigen Kristallen Aminoalkohol-Hydrochloridsalz angeimpft. Innerhalb einer Stunde wurde auf 5 °C abgekühlt und zur Vollendung der Kristallisation wurde weitere 6 Stunden bei 5 °C gerührt.
Das Kristallisat wurde abfiltriert, mit 61,0 g Aceton gewaschen und bei max. 50 °C im Vakuumtrockenschrank (10 mbar) getrocknet. Man erhielt 50,0 g Rohprodukt (Gehalt: ca. 50% Aminoalkohol-Hydrochloridsalz). Dies entsprach einer Ausbeute von 52,0 % bzgl. eingesetztem 2-Azabicyclo[2.2.1]hept-5-en-3-on.

## Patentansprüche

1. Verfahren zur Herstellung von (1S,4R)- oder (1R,4S)-1-Amino-4-(hydroxymethyl)-2-cyclopenten der Formeln 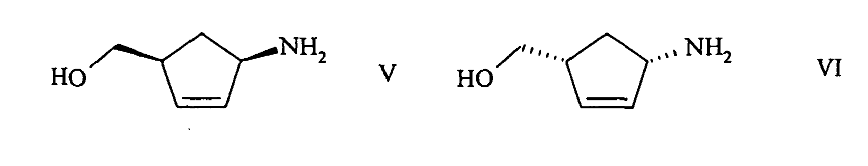 bzw. deren Salzen und / oder von (1S,4R)- oder (1R,4S)-1-Amino-4-(hydroxymethyl)-2-cyclopentenderivaten der allgemeinen Formeln 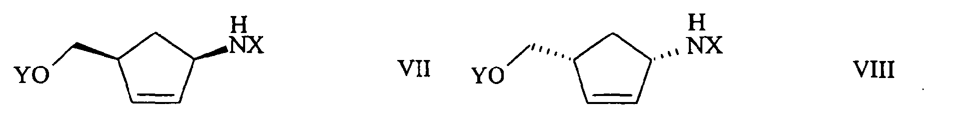 bzw. deren Salzen, worin X und Y gleich oder verschieden sind und eine Acylgruppe oder H bedeuten, ausgenommen X = Y = H, umfassend die biotechnologische Racematspaltung eines racemischen Aminoalkohols der Formel 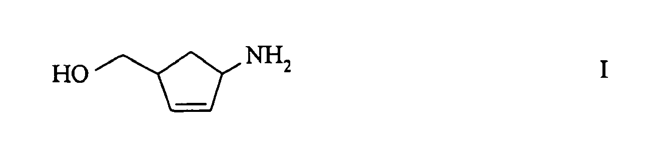 mittels einer Hydrolase in Gegenwart eines Acylierungsmittels, wobei die Hydrolase ausgewählt ist aus der Gruppe bestehend aus Lipasen, Proteasen, Amidasen und Esterasen, und das Acylierungsmittel ausgewählt ist aus der Gruppe bestehend aus Carbonsäureamiden, Carbonsäureanhydriden und Carbonsäureestern.

2. Verfahren nach Anspruch 1, **dadurch gekennzeichnet, dass** die Racematspaltung mittels einer Lipase durchgeführt wird.

3. Verfahren zur Herstellung von (1R,4S)- oder (1S,4R)-1-Amino-4-(hydroxymethyl)-2-cyclopentenderivaten der allgemeinen Formeln 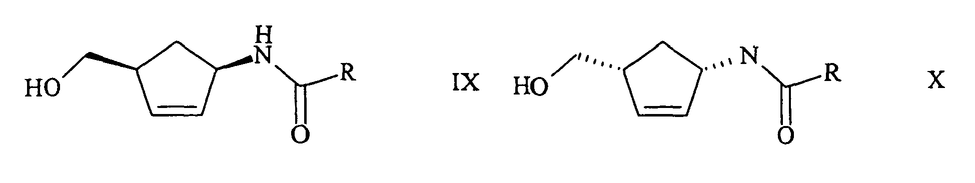 worin R C₁₋₄-Alkyl, C₁₋₄-Alkoxy, Aryl oder Aryloxy bedeutet, **dadurch gekennzeichnet, dass** man in einer ersten Stufe (±)-2-Azabicyclo[2.2.1]hept-5-en-3-on der Formel 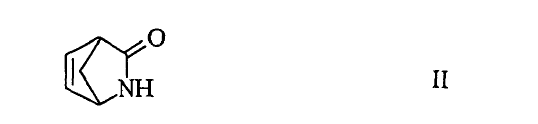 in Form des Racemats oder eines seiner optisch aktiven Isomeren mit einem Metallhydrid zu einem racemischen Aminoalkohol der Formel 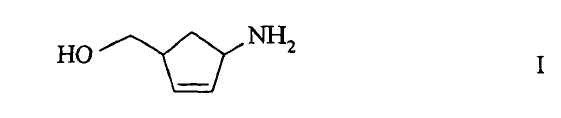 reduziert, diesen in einer zweiten Stufe biotechnologisch mittels einer Hydrolase in Gegenwart eines Acylierungsmittels zum (1S,4R)- oder (1R,4S)-1-Amino-4-(hydroxymethyl)-2-cyclopenten der Formel V oder VI umsetzt und diese zu den Produkten der Formeln IX oder X acyliert, wobei die Hydrolase ausgewählt ist aus der Gruppe bestehend aus Lipasen, Proteasen, Amidasen und Esterasen, und das Acylierungsmittel ausgewählt ist aus der Gruppe bestehend aus Carbonsäureamiden, Carbonsäureanhydriden und Carbonsäureestern.

4. Verfahren nach einem der Ansprüche 1 bis 3, **dadurch gekennzeichnet, dass** das Acylierungsmittel ein Carbonsäureester ist, der ausgewählt ist aus der Gruppe bestehend aus Alkoxycarbonsäureestern, C₁₋₆-Carbonsäureestern, Glykolestern, Dicarbonsäureestern, Cyancarbonsäureestern und cyclischen Estern.

## Claims

1. Process for the preparation of (1S,4R)- or (1R,4S)-1-amino-4-(hydroxymethyl)-2-cyclopentene of the formulae 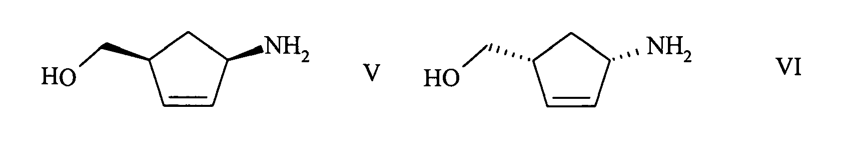 or salts thereof and/or of (1S,4R)- or (1R,4S)-1-amino-4(hydroxymethyl)-2-cyclopentene derivatives of the general formulae 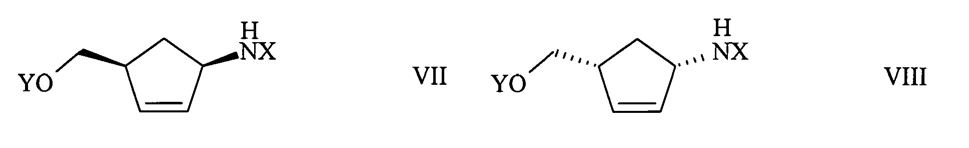 or salts thereof, in which X and Y are identical or different and are an acyl group or H, with the exception of X = Y = H, comprising the racemate resolution of racemic aminoalcohol of the formula 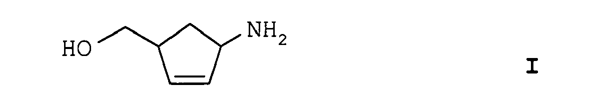 by biotechnological means using a hydrolase in the presence of an acylating agent, wherein the hydrolase is selected from the group consisting of lipases, proteases, amidases and esterases, and the acylating agent is selected from the group consisting of carboxamides, carboxylic anhydrides and carboxylic esters.

2. Process according to Claim 1, **characterized in that** the biotechnological racemate resolution is carried out using a lipase.

3. Process for the preparation of an (1R,4S)- or
(1S,4R)-1-amino-4-(hydroxymethyl)-2-cyclopentene derivative of the general formulae 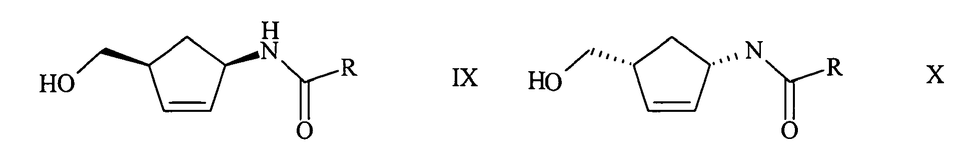 in which R is C₁₋₄-alkyl, C₁₋₄-alkoxy, aryl or aryloxy, **characterized in that**, in a first stage (±)-aza-bicyclo[2.2.1]hept-5-en-3-one of the formula 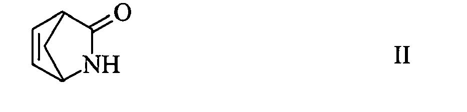 in the form of the racemate or one of its optically active isomers is reduced with a metal hydride into a racemic aminoalcohol of the formula 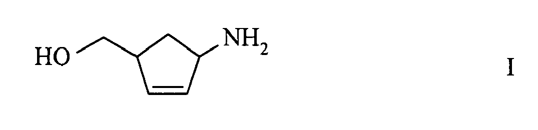 which, in a second stage, is converted by biotechnological means using a hydrolase in the presence of an acylating agent into (1S,4R)- or (1R,4S)-1-amino-4-(hydroxymethyl)-2-cyclopentene of the formula V or VI, which is acylated to give products of the formula IX or X, wherein the hydrolase is selected from the group consisting of lipases, proteases, amidases and esterases, and the acylating agent is selected from the group consisting of carboxamides, carboxylic anhydrides and carboxylic esters.

4. Process according to one of Claims 1 to 3, **characterized in that** the acylating agent is a carboxylic ester, which is selected from the group consisting of alkoxycarboxylic esters, C₁₋₆-carboxylic esters, glycol esters, dicarboxylic esters, cyanocarboxylic esters, such as ethyl cyanoacetate and cyclic esters.

## Revendications

1. Procédé pour la préparation de (1S,4R)- ou (1R,4S)-1-amino-4-(hydroxyméthyl)-2-cyclopentène de formules 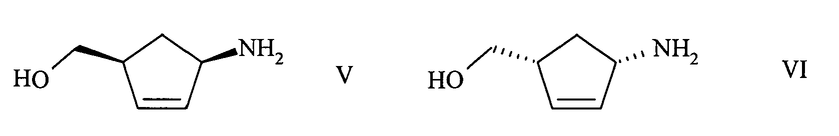 ou de leurs sels et/ou de dérivés de (1S,4R)- ou (1R,4S)-1-amino-4-(hydroxyméthyl)-2-cyclopentène de formules générales 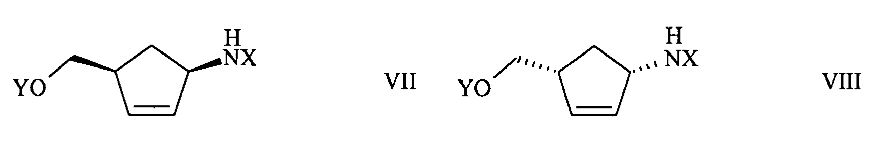 ou de leurs sels, formules dans lesquelles X et Y sont identiques ou différents et représentent un groupe acyle ou H, à l'exception de X = Y = H, comprenant le dédoublement de racémate, par voie biotechnologique, d'un aminoalcool racémique de formule 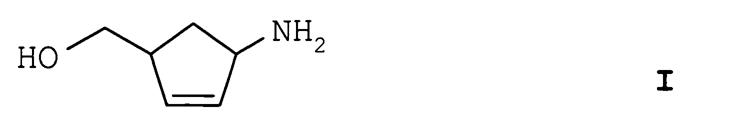 à l'aide d'une hydrolase en présence d'un agent d'acylation, l'hydrolase étant choisie dans le groupe
constitué par les lipases, les protéases, les amidases et les estérases, et l'agent d'acylation étant choisi dans le groupe constitué par des amides d'acides carboxyliques, des anhydrides d'acides carboxyliques et des esters d'acides carboxyliques.

2. Procédé selon la revendication 1, **caractérisé en ce que** le dédoublement du racémate est effectué à l'aide d'une lipase.

3. Procédé pour la préparation de dérivés de (1R,4S)- ou (1S,4R)-1-amino-4-(hydroxyméthyl)-2-cyclo-pentène de formules générales 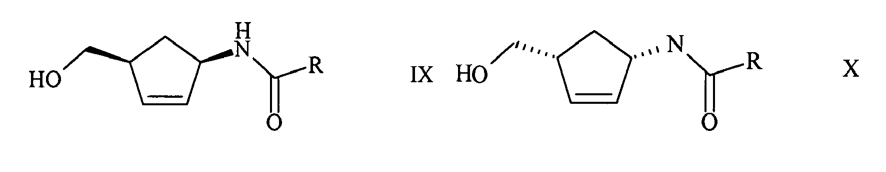 dans lesquelles R représente un groupe alkyle en C₁-C₄, alcoxy en C₁-C₄, aryle ou aryloxy, **caractérisé en ce que** dans une première étape on réduit la (±)-2-aza-bicyclo[2.2.1]hept-5-én-3-one de formule 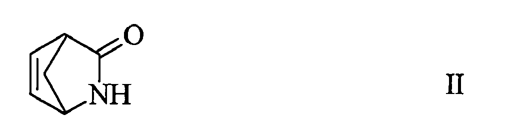 sous forme du racémate ou d'un de ses isomères optiquement actifs, à l'aide d'un hydrure métallique, en un aminoalcool racémique de formule 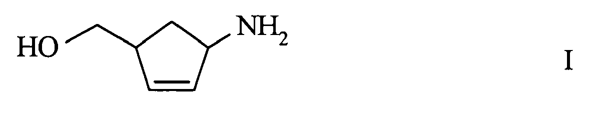 dans une deuxième étape on convertit ce dernier par voie biotechnologique, au moyen d'une hydrolase, en
présence d'un agent d'acylation, en le (1S,4R)- ou (1R,4S)-1-amino-4-(hydroxyméthyl)-2-cyclopentène de formule V ou VI et ces derniers sont acylés en les produits de formule IX ou X, l'hydrolase étant choisie dans le groupe constitué par les lipases, les protéases, les amidases et les estérases, et l'agent d'acylation étant choisi dans le groupe constitué par des amides d'acides carboxyliques, des anhydrides d'acides carboxyliques et des esters d'acides carboxyliques.

4. Procédé selon l'une quelconque des revendications 1 à 3, **caractérisé en ce que** l'agent d'acylation est un ester d'acide carboxylique qui est choisi dans le groupe constitué par des esters d'acides alcoxycarboxyliques, des esters d'acides carboxyliques en C₁-C₆, des esters de glycol, des esters d'acides dicarboxyliques, des esters d'acides cyanocarboxyliques et des esters cycliques.
